# FASCICULE DE BREVET EUROPEEN

(11) **EP 0 650 352 B1**
(45) Date de publication et mention de la délivrance du brevet: **11.09.1996**
(21) Numéro de dépôt: 93915994.3
(22) Date de dépôt: 13.07.1993
(51) Int. Cl.: A61K 9/113, A61K 7/00

(54) **COMPOSITION COSMETIQUE SOUS FORME D'EMULSION TRIPLE EAU/HUILE/EAU A PHASE EXTERNE GELIFIEE**
KOSMETISCHE ZUSAMMENSETZUNG IN FORM EINER DREIFACHEMULSION W/O/W MIT GELIERTERPHASE
COSMETIC COMPOSITION IN THE FORM OF A WATER/OIL/WATER TRIPLE EMULSION WITH GELLED EXTERNAL PHASE

(30) Priorité: 17.07.1992 FR 9208870
(43) Date de publication de la demande: 03.05.1995
(73) Titulaire: L'OREAL, F-75008 Paris (FR)
(72) Inventeur: NADAUD, Jean-François, F-75013 Paris (FR); SEBILLOTTE, Laurence, F-75013 Paris (FR)
(74) Mandataire: Casalonga, Axel
(86) Numéro de dépôt international: FR9300714
(87) Numéro de publication internationale: WO9402120

(56) Documents cités:
- EP-A- 0 174 377
- EP-A- 0 268 164
- EP-A- 0 345 075
- EP-A- 0 446 094
- EP-A- 0 507 693
- FR-A- 2 326 914
- PATENT ABSTRACTS OF JAPAN & JP-A-63 145 659 (LION CORP.) 17 June 1988

## Description

La présente invention concerne une composition cosmétique ou dermatologique se présentant sous forme d'une émulsion triple eau/huile/eau gélifiée, son procédé de préparation et ses applications dans les domaines cosmétique et dermatologique.

Depuis de nombreuses années, on utilise, notamment dans le domaine de la cosmétique, des émulsions dans les produits de traitement cosmétique de la peau. Ces émulsions sont généralement des émulsions huile-dans-eau (H/E) ou eau-dans-huile (E/H).

Des émulsions triples du type E/H/E ou encore H/E/H sont également utilisées en cosmétique ou en dermatologie. De telles émulsions présentent toutefois de nombreux problèmes lors de leur fabrication ou encore des problèmes de stabilité dans le temps, et notamment lorsqu'on introduit dans ces émulsions des substances actives qui peuvent avoir tendance à déstabiliser les émulsions préparées.

La demande de brevet EP-A-0345075 (UNILEVER) décrit des émulsions triples E/H/E dans lesquelles la phase externe continue est gélifiée et qui contiennent un agent de pression osmotique dans la phase aqueuse interne, cet agent attirant l'eau de la phase externe à travers la phase huileuse. Le procédé de préparation de ces émulsions gélifiées consiste à disperser une émulsion eau-dans-huile, dont la phase aqueuse contient un agent de pression osmotique, dans une solution d'un agent gélifiant, du type polysaccharide, gélatine, ou autre protéine. Ces émulsions présentent toutefois des inconvénients. La différence de pression osmotique entre la phase aqueuse interne et la phase aqueuse externe provoque une fuite d'eau de la phase aqueuse externe vers la phase aqueuse interne, donc un gonflement des globules aqueux internes, et une concentration élevée en gélifiant dans la phase aqueuse externe aboutissant à un produit trop gélatineux.

La demande de brevet EP-A-0281394 (RICHARDSON VICKS) se rapporte a une émulsion H/E/silicone. Il s'agit d'une émulsion très particulière, obtenue par introduction d'une émulsion H/E classique dans une phase huileuse siliconée qui constitue la phase huileuse externe. Cette émulsion contient différents agents tensio-actifs jouant le rôle d'émulsifiants dans l'émulsion H/E.

Or, il est connu que l'utilisation d'une quantité importante d'agents tensio-actifs dans une émulsion à usage cosmétique peut provoquer des réactions cutanées du type allergie ou irritation.

Il faut donc chercher à minimiser les quantités d'agents tensio-actifs tout en ne déstabilisant pas l'émulsion.

La demanderesse a réussi à surmonter les difficultés, tant au plan technique qu'au plan cosmétique, liées aux émulsions triples de l'état de la technique et à obtenir une émulsion triple possédant une texture originale, des propriétés cosmétiques intéressantes ainsi qu'une bonne stabilité, sans comporter de tensio-actif dans la phase externe.

L'émulsion triple, selon l'invention, est une émulsion eau/huile/eau gélifiée (E/H/E), dans laquelle l'émulsion primaire eau/huile est distribuée de façon homogène avec des globules de taille comprise entre 0,5 et 50 µm, ce qui lui confère un aspect lisse et brillant. L'émulsion triple selon l'invention est en outre gélifiée, onctueuse, fraîche, douce, non grasse et stable.

L'invention a donc pour objet une émulsion triple présentant les caractéristiques définies ci-après.

Un autre objet de l'invention est constitué par le procédé de préparation d'une telle émulsion.

L'invention a également pour objet l'utilisation cosmétique ou dermatologique d'une telle émulsion.

D'autres objets de l'invention apparaîtront à la lecture de la description et des exemples qui suivent.

L'émulsion triple eau/huile/eau gélifiée conforme à l'invention, est essentiellement caractérisée par le fait qu'elle comporte :
(A) une phase grasse comprenant au moins une cire de point de fusion égal ou supérieur à 60°C, constituant avec une phase aqueuse l'émulsion primaire E/H;
(B) une huile de dilution;
(C) une phase aqueuse externe continue gélifiée comprenant au moins un gélifiant à chaîne grasse du type copolymère acide ou anhydride d'acide carboxylique monoéthylénique en C₃-C₆/ester acrylique à chaîne grasse;
émulsion triple dans laquelle la quantité de phase grasse provenant de l'émulsion E/H est comprise entre 1 et 30% et la quantité de cire(s) de point de fusion égal ou supérieur à 60°C est comprise entre 0,2 et 10%, sur la base du poids total de l'émulsion triple.

La phase aqueuse externe continue gélifiée de l'émulsion triple E/H/E selon l'invention comprend de l'eau et un gélifiant à chaîne grasse du type copolymère d'un acide carboxylique monoéthylénique comportant 3 à 6 atomes de carbone (ou son anhydride) et d'un ester acrylique à chaîne longue.

Ce type de copolymère acrylique hydrosoluble, éventuellement réticulé, qui sera appelé par la suite "gélifiant à chaîne grasse", est décrit dans EP-A-0268164. Dans ce copolymère, la proportion d'acide monomère est, de préférence, de 90 à 98% en poids et la proportion d'ester monomère est, de préférence, de 10 à 2% en poids.

L'acide monomère a pour formule : 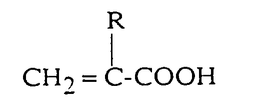 formule dans laquelle R représente H, un halogène, OH, un radical lactone ou lactame, un groupe -C≡N, ou un radical alkyle en C₁-C₃. Les monomères acides préférés sont l'acide acrylique et l'anhydride maléique.

L'ester monomère a pour formule : 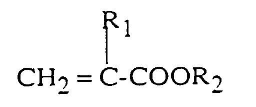 formule dans laquelle :
R₁ est H, méthyle ou éthyle, et
R₂ est un radical alkyle en C₈-C₃₀, ou oxyalkylène en C₈-C₃₀. Les radicaux alkyle en C₁₀-C₂₂ sont préférés. Parmi les esters monomères que l'on préfère, on peut citer : les acrylates et méthacrylates de décyle, lauryle, stéaryle, béhényle et mélissyle.

Les copolymères utilisés selon l'invention sont, au moins pour certains d'entre eux, distribués dans le commerce; ils sont, par exemple, commercialisés sous les dénominations PEMULEN et CARBOPOL 1342 par la Société GOODRICH.

La phase aqueuse externe continue gélifiée peut en outre comprendre d'autres constituants, tels que d'autres gélifiants du type polymères carboxyvinyliques, comme ceux vendus sous les dénominations CARBOPOL 980 ou 942 ou 950, etc., par la Société GOODRICH, ou celui vendu sous la dénomination SYNTHALEN K par la Société SIGMA, polyméthacrylates de glycéryle vendus par la Société GUARDIAN sous la dénomination LUBRAJEL, carraghénanes tels que le produit vendu par la Société SANOFI sous la dénomination SATIAGEL K80 (D. Galactopyrannose sulfate), gommes de xanthane telles que le produit xanthane/polysaccharides comprenant des unités de glucose/mannose/acide glucuronique (40/30/30), vendu par la Société KELCO sous la dénomination KELTROL.

La phase aqueuse externe peut contenir également des glycols et des neutralisants tels que la triéthanolamine ou l'hydroxyde de sodium. Elle peut aussi contenir des agents conservateurs, des colorants, des parfums, des actifs, des filtres solaires, des hydratants tels que la glycérine et des agents d'onctuosité tels que le produit SEPIGEL 305 vendu par la Société SEPPIC.

L'émulsion primaire eau-dans-huile (E/H) utilisée dans' l'émulsion triple E/H/E selon l'invention, comprend un taux important. supérieur à 35 % en poids, de phase grasse contenant au moins une cire de point de fusion égal ou supérieur à 60°C et une phase aqueuse, ainsi qu'un ou plusieurs émulsionnants.

Les émulsionnants contenus dans l'émulsion primaire E/H selon l'invention, peuvent être choisis parmi les tensio-actifs suivants :
- *Les tensio-actifs anioniques* tels que les sels d'acides gras (par exemple sels métalliques ou sels organiques tels que les sels d'amines); ces acides gras ont par exemple 12 à 18 atomes de carbone et peuvent comporter une double liaison comme dans le cas de l'acide oléïque. On peut citer par exemple le NOREMULSOL G5 vendu par la Société VERLEY (distéarate d'aluminium/acide stéarique/sulfate de sodium/39/56/5), l'ALLUGEL 44 M vendu par la Société BARLOCHER (stéarate d'aluminium).

Les autres tensio-actifs anioniques sont les sels alcalins ou sels de bases organiques des acides alkylsulfuriques et alkylsulfoniques ayant 12 à 18 atomes de carbone, des acides alkylarylsulfoniques dont la chaîne alkyle contient de 6 à 8 atomes de carbone, les éthers-sulfates, en particulier les produits de sulfatation des alcools gras et alkylphénols polyalcoxylés dans lesquels la chaîne aliphatique comporte de 6 à 20 atomes de carbone et la chaîne polyalcoxylée de 1 à 30 motifs oxyalkylènes dont oxyéthylène, oxypropylène ou oxybutylène.
- *Les tensio-actifs non-ioniques* sont principalement des tensio-actifs polyalcoxylés et/ou polyglycérolés. Ce sont notamment des dérivés polyoxyéthylénés. éventuellement polyoxypropylénés. Font partie de cette catégorie les acides gras ou les amides d'acides gras polyalcoxylés et/ou polyglycérolés. Ce sont des matières premières vendues par exemple sous la dénomination MYRJ par la Société ATLAS ou PROTEGIN vendues par la Société GOLDSCHMIDT. Lorsque le polyol est le sorbitol, on peut citer les produits vendus sous la dénomination TWEEN ou ARLACEL par la Société ICI. Les alcools gras ou les alkylphénols polyalcoxylés et/ou polyglycérolés sont vendus par la Société ATLAS sous la dénomination BRIJ.

Les autres tensio-actifs non-ioniques sont les alcanediols ou alcènediols -1,2 ou -1,3 polyalcoxylés, les alkyléthers d'alcanediols ou alcènediols - 1,2 ou 1,3 polyalcoxylés et/ou polyglycérolés.

Les acides ou alcools gras, éventuellement insaturés, ont 12 à 24 atomes de carbone, la chaîne alkyle des alkylphénols a 6 à 16 atomes de carbone, les alcanediols ou alcènediols ont de 9 à 24 atomes de carbone, la chaîne alkyle des alkyléthers a de 4 à 20 atomes de carbone et le nombre de motifs oxyalkylène ou de motifs (CH₂CHOH CH₂O) peut aller de 2 à 40.

On peut y ajouter des *co-émulsionnants* pouvant être des lécithines hydrogénées, ou non, ou des *stabilisants* comme les sels minéraux tels que le sulfate de magnésium le chlorure de sodium.

*Les cires de température de fusion supérieure à 60°C* sont par exemple des cires fossiles dont l'ozokérite, la cire de montan, des cires d'origine animale dont la cire d'abeille ou des cires d'origine végétale dont la cire de candellila et la cire de carnauba.

On peut y ajouter *des cires de température de fusion inférieure à 60°C* telles que les cires minérales dont les cires microcristallines, la paraffine, la vaseline, les cires d'origine animale dont le spermaceti, la lanoline et ses dérivés (alcool de lanoline, lanoline hydrogénée, lanoline hydroxylée, lanoline acétylée, acides gras de lanoline, alcool de lanoline acétylée).

On peut également ajouter dans la phase grasse de l'émulsion primaire, *des huiles hydrogénées* concrètes à 25°C telles que l'huile de ricin hydrogénée, le suif hydrogéné, et les esters gras concrets à 25°C tels que le monomyristate de propylèneglycol, le myristate de myristyle.

*Les huiles* susceptibles d'être utilisées dans l'émulsion primaire sont les huiles minérales telles que l'huile de paraffine, l'huile de vaseline et les huiles minérales ayant un point d'ébullition entre 300 et 400°C; les huiles d'origine animale telles que le perhydrosqualène; les huiles végétales telles que l'huile d'amande douce, l'huile de calophyllum, l'huile de palme l'huile de noyau d'abricot, l'huile d'avocat, l'huile de jojoba, l'huile d'olive, l'huile de ricin, les huiles de germes de céréales; les huiles synthétiques comme les esters d'acides gras tels que l'huile de purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylèneglycol; les esters dérivés d'acide lanolique tels que le lanolate d'isopropyle, le lanolate d'isocétyle; d'autres huiles telles que les acétylglycérides, les octanoates et décanoates d'alcool et de polyalcools tels que ceux de glycol et de glycérol les ricinoléates d'alcools et de polyalcools tels que ceux de cétyle.

L'émulsion primaire peut contenir aussi *des alcools gras* tels que l'alcool oléïque, l'alcool isostéarique, l'alcool cétylique, l'alcool stéarylique l'octyldodécanol.

La phase grasse de l'émulsion primaire E/H peut en outre contenir des additifs lipophiles habituellement utilisés en cosmétique, par exemple des substances actives et des filtres solaires.

La phase aqueuse de l'émulsion primaire E/H comprend de l'eau et peut contenir un hydratant tel que la glycérine, un polyol tel que le propylèneglycol, l'hexylèneglycol, le dipropylèneglycol, le 1,3-butylèneglycol. Elle peut aussi contenir les additifs habituels tels que conservateurs, parfums, colorants, ainsi que des actifs et filtres solaires.

La demanderesse a constaté que l'ajout dans l'émulsion primaire E/H de composés comportant des chaînons organofluorés et des chaînons hydrocarbonés permet de diminuer l'effet collant provoqué par le gélifiant à chaîne grasse contenu dans la phase aqueuse externe, et permet d'apporter plus de douceur et d'onctuosité au produit fini.

Ces composés comportant des chaînons organofluorés et des chaînons hydrocarbonés, ont une structure chimique comportant un squelette carboné où certains atomes d'hydrogène ont été substitués par des atomes de fluor, le squelette carboné pouvant comporter un ou plusieurs hétéroatomes et un ou plusieurs groupements organiques fonctionnels.

Pour les composés comportant des chaînons organofluorés et des chaînons hydrocarbonés, on définit le taux de substitution des atomes d'hydrogène par des atomes de fluor, sous la forme du rapport : nombre d'atomes de fluor/(nombre d'atomes de fluor + nombre d'atomes d'hydrogène) où seuls les atomes d'hydrogène liés aux atomes de carbone du squelette sont pris en compte. Les composés comportant des chaînons organofluorés et des chaînons hydrocarbonés utilisés dans les émulsions selon l'invention, comportent au moins un groupement hydrocarboné dans la molécule.

Ces composés comportant des chaînons organofluorés utilisés selon l'invention, ont de préférence un taux de substitution compris entre 0,5 et 95%. De façon préférée, ce taux est supérieur à 10% et inférieur à 80%.

Les composés comportant des chaînons organofluorés et des chaînons hydrocarbonés utilisés selon la présente invention, ont la formule suivante :

(R_{F})ₓ - (A)_{y} - (R_{H})_{z}

dans laquelle :
x représente 1, 2 ou 3,
y représente 0 ou 1,
z représente 0, 1, 2 ou 3,
à la condition que y et z ne soient pas simultanément 0, et que lorsque z est 0, x est 2 ou 3.
R_{F} représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote, et/ou substituée par des atomes d'hydrogène ou d'autres atomes d'halogène, à la condition que, pour deux atomes de carbone de la chaîne, ne soit pas présent plus d'un de ces substituants autres que le fluor.
R_{H} représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote.
A représente un radical di-, tri- ou quadrivalent, tel que : 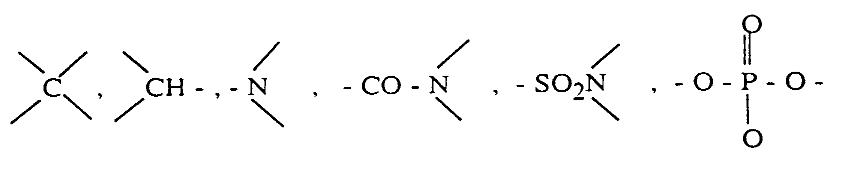 ainsi que les structures cycliques, aliphatiques ou aromatiques comprenant un tel radical, ou des insaturations éthyléniques.

Par l'expression "fonctionnalisé", on entend selon l'invention, une substitution du squelette intercalaire, terminale ou pendante, par au moins un groupement organique fonctionnel comme une fonction alcool, thiol, acide, carbonyle, sulfoxyde, ester, amide, amine, phosphate, éthylénique, acétylénique, et énamine ou sulfonamide.

Par insaturation éthylénique, on entend par exemple : 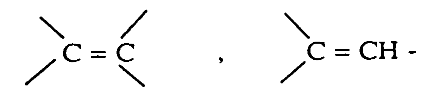 ou - CH = CH -

De préférence, R_{H} représente un radical alkyle linéaire ou ramifié en C₁-C₂₂ ou un mélange de radicaux alkyle linéaires ou ramifiés en C₁-C₂₂, un radical aryle en C₆-C₁₀ ou un radical aralkyle en C₇-C₁₅.

De préférence, R_{F} représente un radical perfluoroalkyle ayant de 4 à 22 atomes de carbone.

A titre illustratif, on peut citer les composés possédant des groupes perfluorocarbonés et des groupes hydrocarbonés, le nombre total d'atomes de carbone étant compris entre 10 et 30, le nombre d'atomes de carbone des groupes hydrocarbonés étant égal ou supérieur à deux fois le nombre d'atomes de carbone des groupes perfluorocarbonés tels qu'ils sont décrits dans le document JP 63-002916.

De même, à titre illustratif, on peut citer les composés organofluorés hydrocarbonés dont la structure générale est définie par la formule :

R₁ - (CH₂)ₙ - X - [C₃H₅ (OH)] - (Y)ₓ - R₂

où C₃H₅ (OH) représente : 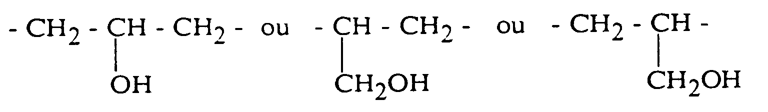
R₁ représente un radical alkyle perfluoré, linéaire ou ramifié en C₄-C₂₀ ou un mélange de radicaux perfluorés, linéaires ou ramifiés en C₄-C₂₀,
R₂ représente un radical alkyle linéaire ou ramifié en C₁-C₂₂ ou un mélange de radicaux alkyle, linéaires ou ramifiés en C₁-C₂₂ ou un radical aryle en C₆-C₁₀ ou aralkyle en C₇-C₁₅,
X et Y, identiques ou différents, représentent : - O - , - S -, 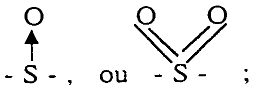 sous réserve que X et Y ne représentent pas simultanément 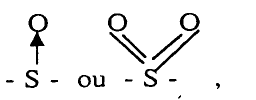
n est compris entre 0 et 4, et
x représente 0 ou 1.

Ces composés utilisés selon l'invention sont décrits dans FR-A 2.684.668 et EP-A-166.696.

Par ailleurs, on peut également utiliser, selon l'invention, les composés de formule :

R_{F} - (CH₂)ₙ - X - [C₃H₅ (OH)] - Y - (CH₂)ₘ - R'_{F}

dans laquelle C₃H₅ (OH) représente les structures : 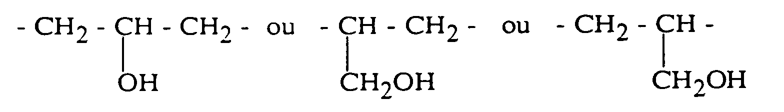
R_{F} et R'_{F}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4;
X et Y, identiques, sont - O - ou - S - .

Ces composés sont décrits dans DE-2.702.607, JP 89-193.236, JP 92-275.268 et US-3.893.984.

On peut aussi utiliser, selon l'invention, les composés décrits dans le document US-3.952.066, de formule : 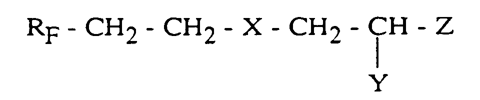 où Y est OH, et

Z est 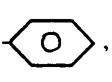 - CH₃, - CH₂OH, - CH₂ O COCH₃
   ou bien Y est - CH₂OH et Z est - O-COCH₃
X représente - O-, - S- , 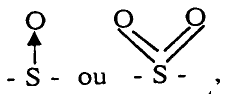 et
R_{F} représente un radical alkyle perfluoré, linéaire ou ramifié, en C₄-C₂₀, ou un mélange de radicaux alkyle perfluorés, linéaires ou ramifiés, en C₄-C₂₀ ;
ou bien les composés décrits dans le document DE 2.052.579, de formule :

R_{F} - CH = CH - CH₂ - O - CH₂ - [C₂H₄ - OW]

où

C₂H₄OW désigne : 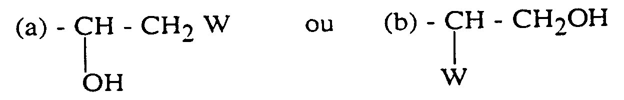
W désignant : -OR, -SR, -COOR, 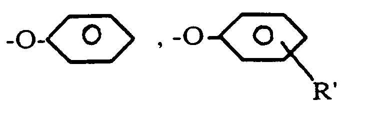
R désigne un radical alkyle en C₁-C₁₈, linéaire ou ramifié,
R' désigne -CH₃ ou -OH, en position ortho ou para, et
R_{F} représente un radical alkyle perfluoré, linéaire ou ramifié, en C₄-C₂₀, ou un mélange de radicaux alkyle perfluorés, linéaires ou ramifiés, en C₄-C₂₀.

On dilue l'émulsion primaire avec une huile citée ci-dessus avant de la disperser dans la phase aqueuse externe continue gélifiée, pour obtenir l'émulsion triple finale stable.

En procédant de la sorte, la taille des globules de l'émulsion triple est comprise entre 0,5 et 50 µm.

L'émulsion triple de la présente invention contient 0,02 à 0,7% en poids d'émulsionnant(s) E/H, 0,2 à 10% en poids de cire(s) de point de fusion égal ou supérieur à 60°C, 5 à 20% en poids d'huile(s), 0,5 à 20% en poids de cire(s) à point de fusion inférieur à 60°C, 0,1 à 3% en poids de gélifiant à chaîne grasse, des neutralisants (triéthanolamine, hydroxyde de sodium), ainsi que les coémulsionnants de l'émulsion primaire indiqués ci-dessus, et éventuellement des substances actives cosmétologiques ou dermatologiques, ainsi que d'autres adjuvants habituellement utilisés en cosmétique tels que des agents conservateurs, des colorants, des parfums, des hydratants, des filtres solaires, le complément étant constitué par de l'eau.

L'invention a également pour objet le procédé de préparation d'une émulsion triple selon l'invention.

On prépare, dans un premier temps, l'émulsion primaire eau-dans-huile en ajoutant une phase aqueuse à la phase grasse pour l'obtention d'une émulsion E/H.

Dans un deuxième temps, on dilue l'émulsion primaire ainsi obtenue, avant sa dispersion dans la phase aqueuse gélifiée, avec une huile telle que citée ci-dessus qui peut être identique à ou différente de celle utilisée dans l'émulsion primaire.

Dans un troisième temps, on réalise l'émulsion triple par addition de l'émulsion primaire ainsi diluée avec une huile, à une seconde phase aqueuse gélifiée qui constitue la phase aqueuse externe de l'émulsion.

Comme déjà indiqué ci-dessus, les compositions sous forme d'émulsion triple conformes à l'invention, présentent des propriétés cosmétiques particulièrement remarquables, notamment au niveau du toucher et de l'aspect, ce qui permet de les utiliser comme bases pour appliquer les substances actives cosmétiques sur la peau.

L'introduction dans la phase aqueuse interne et/ou externe et/ou dans la phase huileuse de substances actives, permet de nombreuses utilisations d'une telle émulsion triple.

Ces émulsions peuvent notamment être utilisées dans des produits de soin du visage pour peaux sèches ou peaux grasses. Pour la réalisation de produits pour peaux sèches, on peut introduire dans l'une quelconque des deux phases aqueuses, des substances actives hydrosolubles hydratantes telles que par exemple la glycérine, le propylèneglycol, le sorbitol, la proline, l'acide pyrrolidone carboxylique et ses dérivés, l'urée, le collagène hydrolysé, le gel d'Aloe Vera, l'acide hyaluronique et ses dérivés, le hyaluronate de diméthylsilanol et l'allantoïne.

Des produits de traitement pour peaux grasses sont obtenus en introduisant dans l'une quelconque des deux phases aqueuses, des substances actives hydrosolubles telles que la provitamine B5 qui est utilisée comme adoucissant ou un antibactérien tel que le transthiolane diol 3,4-S-dioxyde.

Les émulsions selon l'invention peuvent également être utilisées comme produits démaquillants ou de nettoyage pour le visage sous forme de crèmes, de laits ou de masques par exemple, ou comme produits de maquillage par incorporation de charges ou de pigments.

Ces émulsions peuvent aussi être utilisées comme produits solaires par introduction de filtres.

Comme filtres solaires hydrosolubles pouvant être introduits dans la phase aqueuse interne et/ou externe, on peut utiliser par exemple l'acide 2-hydroxy 4-méthoxy benzophénone 5-sulfonique vendu sous la dénomination UVINUL MS 40 par la Société BASF. Comme filtres solaires liposolubles à introduire dans la phase huileuse, on peut utiliser par exemple le paraméthoxycinamate de 2-éthylhexyle vendu sous la dénomination PARSOL MCX par la Société GIVAUDAN ou la 2-hydroxy 4-méthoxybenzophénone vendue sous la dénomination UVINUL M 40 par la Société BASF.

Les émulsions selon l'invention peuvent également être utilisées dans la préparation de produits après-solaires contenant par exemple en tant qu'agents actifs apaisants, la vitamine F et les hydratants cités plus haut.

On peut aussi, en introduisant des substances actives amincissantes hydrosolubles ou liposolubles respectivement dans l'une des deux phases aqueuses ou dans la phase grasse, obtenir des produits amincissants.

Parmi les substances actives amincissantes hydrosolubles, on peut citer les dérivés de xanthine tels que la caféine, la théobromine, la théophylline, la L-carnitine, le chlorhydrate de diméthylaminoéthyl théophylline, les dérivés de silicium du type méthylsilanol théophyllinacétatealginate ou des dérivés végétaux tels que des extraits hydroglycoliques de lierre grimpant, d'algue brune, de pensée sauvage fraîche. Parmi les substances actives amincissantes liposolubles, on peut citer le nicotinate de DL-alpha-tocophérol, l'extrait huileux de racine de ginseng (Panax ginseng), l'extrait huileux de lierre grimpant (Hedera Helix), l'extrait huileux de fleurs sèches d'amica (Arnica Montana L), l'extrait huileux d'algue (Fucus Vesiculosus).

On peut également obtenir des produits de soin corporel en introduisant des hydratants cités précédemment et des huiles végétales ou minérales, ainsi que des produits capillaires utilisés par exemple pour lisser les cheveux en introduisant des filtres solaires pour protéger les cheveux du rayonnement UV.

Les émulsions triples de l'invention peuvent également être utilisées comme produits pour jambes lourdes contenant comme actifs hydrosolubles le ginkgo biloba, le mélilot ou le ruscus et des huiles classiques comme émollients.

Les exemples suivants sont destinés à illustrer l'invention sans pour autant présenter un caractère limitatif.

### EXEMPLES DE FORMULATION

### EXEMPLE 1

### CREME DE SOIN DU VISAGE (E/H/E)

| **PHASE A (E/H)** | |
|---|---|
| * HUILE DE VASELINE | 2,7 g |
| * VASELINE BLANCHE | 2 g |
| - LANOLINE (SOCIETE STELLA) | 0,8 g |
| - CIRE D'ABEILLE PURE | 1,05 g |
| * NOREMULSOL G5 ^{R} (Tensio-actif anionique) (SOCIETE VERLEY) | 0,1 g |
| - CHOLESTEROL | 0,08 g |
| - LECITHINE | 0,06 g |
| - LECITHINE HYDROGENEE | 0,07 g |
| - EAU | 3,14 g |

| **PHASE B** | |
|---|---|
| * PURCELLIN LIQUIDE HUILE 2/066210 ^{R} (SOCIETE DRAGOCO) | 10 g |

| **PHASE C** | |
|---|---|
| * CARBOPOL 1342 ^{R} (SOCIETE GOODRICH) | 0,6 g |
| * TRIETHANOLAMINE | 0,6 g |
| * GLYCERINE | 3 g |
| - CONSERVATEURS,PARFUMS, | |
| - COLORANTS qs | |
| - EAU DISTILLEE qsp | 100 g |

| | |
|---|---|
| * cf annexe décrivant les matières premières. | |

Pour préparer l'émulsion triple, on prépare la phase A, puis on y ajoute la phase B et on disperse le mélange obtenu dans la phase C.

Un panel de 34 femmes a jugé la crème agréable, confortable, de texture originale, peu grasse et douce.

### EXEMPLE 2

### LAIT DE SOIN DU CORPS (E/H/E)

| **PHASE A (E/H)** | |
|---|---|
| * HUILE DE VASELINE | 1,61 g |
| * VASELINE BLANCHE | 1,2 g |
| - OZOKERITE | 0,63 g |
| * NOREMULSOL G5 ^{R} (Tensio-actif anionique) | 0,06 g |
| - LECITHINE | 0,04 g |
| - LECITHINE HYDROGENEE | 0,04 g |
| - EAU | 3,21 g |

| **PHASE B** | |
|---|---|
| * HUILE DE VASELINE | 10 g |

| **PHASE C** | |
|---|---|
| * PEMULEN TR2 ^{R} (SOCIETE GOODRICH) | 0,5 g |
| * GLYCERINE | 5 g |
| * TRIETHANOLAMINE | 0,5 g |
| - CONSERVATEURS,PARFUMS, | |
| - COLORANTS qs | |
| - EAU DISTILLEE qsp | 100 g |

Pour préparer l'émulsion triple, on prépare la phase A, on la dilue dans la phase B, puis on la disperse dans la phase C.

L'émulsion triple se présente sous forme d'un lait fluide, brillant, onctueux et frais.

### EXEMPLE 3

### CREME DE SOIN DU CORPS AMINCISSANTE (E/H/E)

| **PHASE A (E/H)** | |
|---|---|
| * HUILE DE VASELINE | 1,7 g |
| - OZOKERITE | 1,2 g |
| - CIRE DE CARNAUBA | 1 g |
| * VASELINE BLANCHE | 2,5 g |
| * NOREMULSOL G5 ^{R} (Tensio-actif anionique) | 0,12 g |
| - LECITHINE | 0,18 g |
| - EAU | 3,3 g |

| **PHASE B** | |
|---|---|
| - HUILE D'AMANDE DOUCE | 10 g |

| **PHASE C** | |
|---|---|
| * PEMULEN TR1 ^{R} (SOCIETE GOODRICH) | 0,6 g |
| * CAFEINE | 0,5 g |
| * TRIETHANOLAMINE | 0,6 g |
| - CONSERVATEURS,COLORANTS qs | |
| - PARFUMS,ANTIOXYDANTS qs | |
| - EAU DISTILLEE qsp | 100 g |

Pour préparer l'émulsion triple, on prépare la phase A, on la dilue dans la phase B, puis on disperse la préparation obtenue dans la phase C.

On obtient une crème fraîche qui s'étale et pénètre bien et qui permet le massage.

### EXEMPLE 4

### MASQUE POUR LE SOIN DU VISAGE (E/H/E)

| **PHASE A (E/H)** | |
|---|---|
| - PROTEGIN X (Tensio-actif non-ionique) (SOCIETE GOLDSCHMIDT) | 1,9 g |
| - ALLUGEL 44 M (Tensio-actif anionique) (SOCIETE BARLOCHER) | 0,05 g |
| - OZOKERITE | 1,4 g |
| * VASELINE BLANCHE | 2,5 g |
| - SULFATE DE MAGNESIUM | 0,04 g |
| * GLYCERINE | 0,3 g |
| - EAU | 3,81 g |

| **PHASE B** | |
|---|---|
| - HUILE DE NOYAU D'ABRICOT | 10 g |

| **PHASE C** | |
|---|---|
| * CARBOPOL 1342 ^{R} (SOCIETE GOODRICH) | 0,7 g |
| * GLYCERINE | 3 g |
| - HYDROXYDE DE SODIUM | 0,6 g |
| - CONSERVATEURS,COLORANTS qs | |
| - PARFUMS,ANTIOXYDANTS qs | |
| - EAU DISTILLEE qsp | 100 g |

Pour préparer l'émulsion triple, on prépare la phase A, on la dilue dans la phase B, puis on disperse l'ensemble dans la phase C.

On obtient une crème gélifiée qui, en application sur le visage, donne un masque hydratant que l'on peut enlever au bout de 5 à 10 minutes.

### EXEMPLE 5

### CREME DE SOIN POUR LE CORPS (E/H/E)

| **PHASE A (E/H)** | |
|---|---|
| - 1-(2'-F-hexyléthylthio)3-(2''-éthylhexyloxy)2-propanol | 1 g |
| * HUILE DE VASELINE | 2,7 g |
| * VASELINE BLANCHE | 2 g |
| - LANOLINE (SOCIETE STELLA) | 0,8 g |
| - CIRE D'ABEILLE PURE | 1,05 g |
| * NOREMULSOL G5^{R} (SOCIETE VERLEY) | 0,1 g |
| - CHOLESTEROL | 0,08 g |
| - LECITHINE | 0,06 g |
| - LECITHINE HYDROGENEE | 0,07 g |
| - EAU | 2,14 |

| **PHASE B** | |
|---|---|
| * PURCELLIN LIQUIDE HUILE 2/066210^{R} (SOCIETE DRAGOCO) | 10 g |

| **PHASE C** | |
|---|---|
| * CARBOPOL 1342 ^{R} (SOCIETE GOODRICH) | 0,6 g |
| * TRIETHANOLAMINE | 0,6 g |
| * GLYCERINE | 3 g |
| - CONSERVATEUR(S),PARFUM(S) | |
| - COLORANT(S) qs | |
| - EAU qsp | 100 g |

### EXEMPLE 6

### CREME DE SOIN POUR LE VISAGE (E/H/E)

| **PHASE A (E/H)** | |
|---|---|
| * HUILE DE VASELINE | 1,7 g |
| - OZOKERITE | 1,2 g |
| - CIRE DE CARNAUBA | 1 g |
| * VASELINE BLANCHE | 2,5 g |
| * NOREMULSOL G5^{R} (SOCIETE VERLEY) | 0,12 g |
| - LECITHINE | 0,18 g |
| - EAU | 3,3 g |

| **PHASE B** | |
|---|---|
| - HUILE D'AMANDE DOUCE | 10 g |

| **PHASE C** | |
|---|---|
| * PEMULEN TR1^{R} (SOCIETE GOODRICH) | 0,6 g |
| * CAFEINE | 0,5 g |
| * TRIETHANOLAMINE | 0,6 g |
| - CONSERVATEUR(S),COLORANT(S) qs | |
| - PARFUM(S),ANTIOXYDANTS(S) qs | |
| - POLYACRYLAMIDE/ISOPARAFFINE C₁₃-C₁₄/LAURETH 7 (SEPIGEL 305 de la SOCIETE SEPPIC) | 1 g |
| - EAU qsp | 100 g |

### EXEMPLE 7

### CREME DE SOIN POUR LE COU (E/H/E)

| **PHASE A (E/H)** | |
|---|---|
| * HUILE DE VASELINE | 1,61 g |
| * VASELINE BLANCHE | 1,2 g |
| - OZOKERITE | 0,63 g |
| * NOREMULSOL G5^{R} (SOCIETE VERLEY) | 0,06 g |
| - LECITHINE | 0,04 g |
| - LECITHINE HYDROGENEE | 0,04 g |
| - EAU | 3,61 g |

| **PHASE B** | |
|---|---|
| * HUILE DE VASELINE | 10 g |

| **PHASE C** | |
|---|---|
| * CARBOPOL 1342 ^{R} (SOCIETE GOODRICH) | 0,5 g |
| - CARBOMER (dénomination CTFA) (SYNTHALEN K^{R} de la SOCIETE SIGMA) | 0,5 g |
| * GLYCERINE | 5 g |
| * TRIETHANOLAMINE | 1 g |
| - CONSERVATEUR(S),PARFUM(S) qs | |
| - COLORANT(S) qs | |
| - EAU qsp | 100 g |

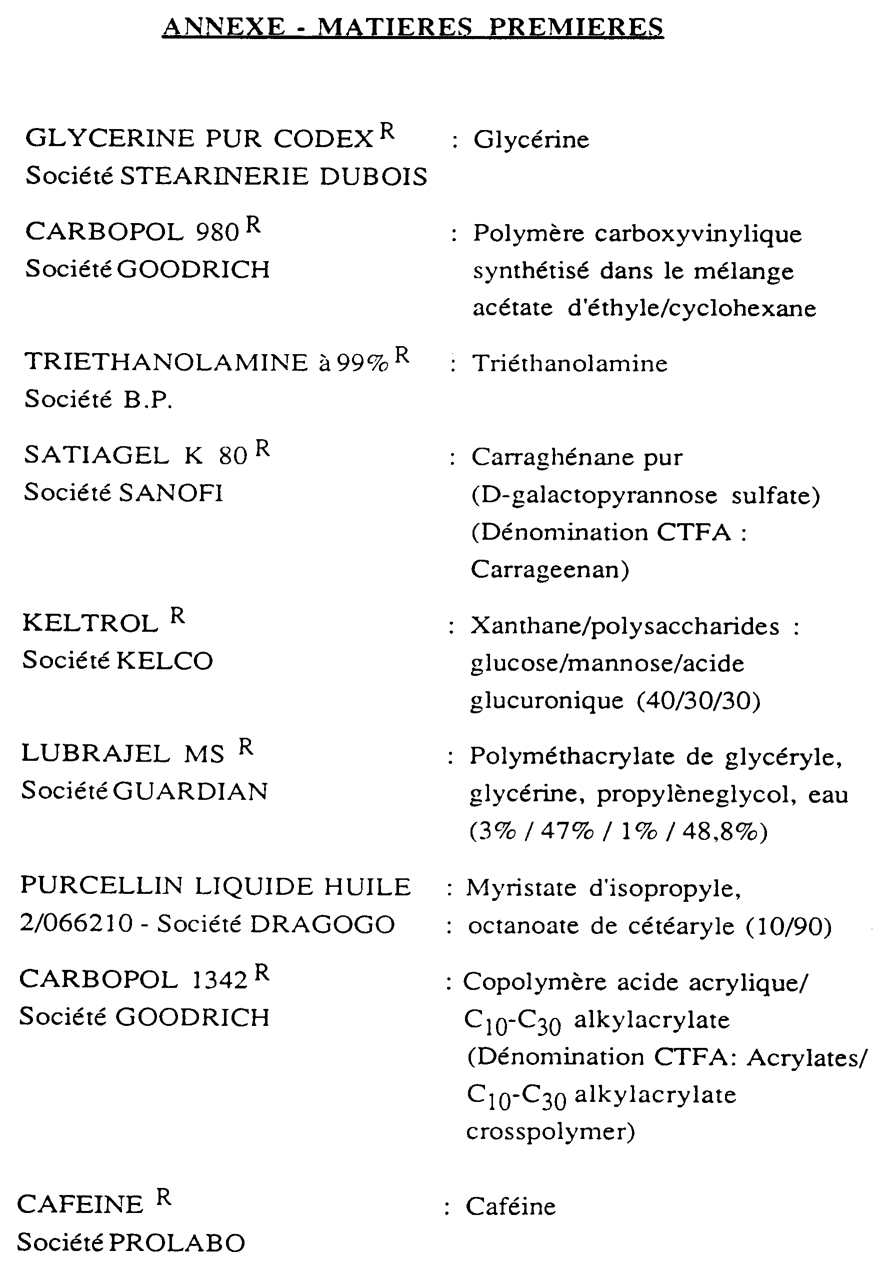

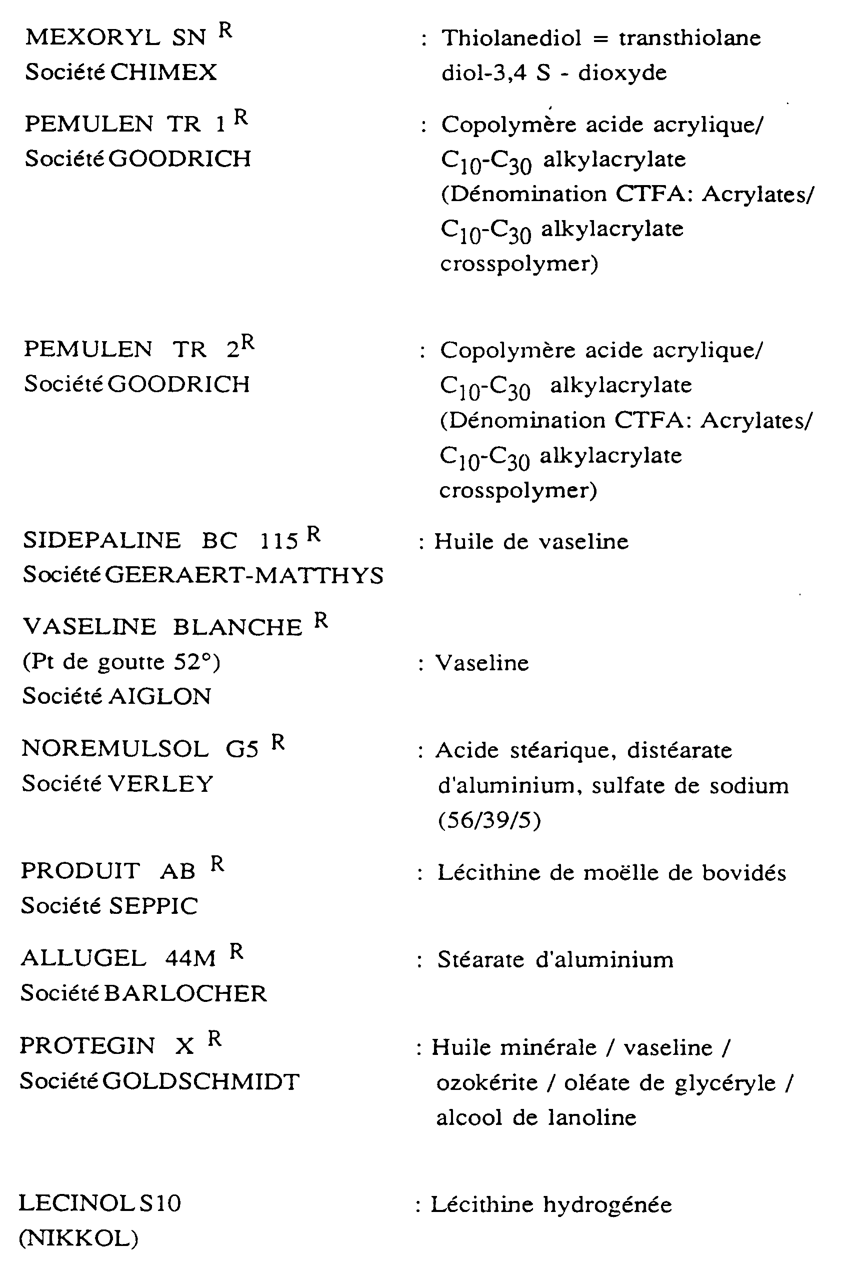

## Revendications

1. Emulsion triple eau/huile/eau gélifiée, caractérisée par le fait qu'elle comporte :
(A) une phase grasse comprenant au moins une cire de point de fusion égal ou supérieur à 60°C, constituant avec une phase aqueuse, l'émulsion primaire E/H;
(B) une huile de dilution;
(C) une phase aqueuse externe continue gélifiée, comprenant au moins un gélifiant à chaîne grasse du type copolymère acide ou anhydride d'acide carboxylique monoéthylénique en C₃-C₆/ester acrylique à chaîne grasse;
émulsion triple dans laquelle la quantité de phase grasse provenant de l'émulsion E/H est comprise entre 1 et 30% et la quantité de cire(s) de point de fusion égal ou supérieur à 60°C, est comprise entre 0,2 et 10%, sur la base du poids total de l'émulsion triple.

2. Emulsion triple selon la revendication 1, caractérisée par le fait que la quantité de phase grasse dans l'émulsion primaire E/H est supérieure à 35% en poids.

3. Emulsion triple selon la revendication 1 ou 2, caractérisée par le fait que la phase aqueuse externe gélifiée comporte, en outre, un autre gélifiant choisi parmi les polymères carboxyvinyliques, les polyméthacrylates de glycéryle les carraghénanes et les gommes de xanthane.

4. Emulsion triple selon l'une quelconque des revendications 1 à 3, caractérisée par le fait que la phase aqueuse externe gélifiée contient en outre des glycols, des neutralisants, des conservateurs, des colorants, des parfums, des substances actives, des filtres solaires, des hydratants et des agents d'onctuosité.

5. Emulsion triple selon l'une quelconque des revendications 1 à 4, caractérisée par le fait que la phase grasse de l'émulsion primaire E/H comprend, en outre, des cires de point de fusion inférieur à 60°C telles que des cires minérales, des cires d'origine animale, la lanoline ou ses dérivés, des huiles hydrogénées concrètes à 25°C, des huiles minérales, des huiles d'origine animale, des huiles végétales, des esters d'acides gras, des esters dérivés d'acide lanolique, des alcools gras.

6. Emulsion triple selon l'une quelconque des revendications 1 à 5, caractérisée par le fait que la phase aqueuse de l'émulsion primaire comprend, outre de l'eau, des hydratants, des polyols, des substances actives, des conservateurs, des filtres solaires, des parfums et des colorants.

7. Emulsion triple selon l'une quelconque des revendications 1 à 6, caractérisée par le fait que l'émulsion primaire E/H comprend au moins un émulsionnant choisi parmi les tensio-actifs anioniques, tels que les sels métalliques ou les sels organiques d'acides gras contenant 12 à 18 atomes de carbone, les sels alcalins ou les sels de bases organiques des acides alkylsulfuriques et alkylsulfoniques contenant 12 à 18 atomes de carbone, des acides alkylarylsulfoniques dont la chaîne alkyle contient 6 à 8 atomes de carbone, les éthersulfates, et les tensio-actifs non-ioniques polyalcoxylés et/ou polyglycérolés.

8. Emulsion triple selon l'une quelconque des revendications 1 à 7, caractérisée par le fait que l'émulsion primaire E/H contient un co-émulsionnant choisi parmi les lécithines hydrogénées ou non, et des sels minéraux stabilisants.

9. Emulsion triple selon l'une quelconque des revendications 1 à 8, caractérisée par le fait que l'émulsion primaire E/H contient au moins un composé comportant des chaînons organofluorés et des chaînons hydrocarbonés.

10. Emulsion triple selon la revendication 9, caractérisée par le fait que le composé comportant des chaînons organofluorés et des chaînons hydrocarbonés a un taux de substitution compris entre 0,5 et 95%.

11. Emulsion triple selon l'une quelconque des revendications 1 à 10, caractérisée par le fait que l'huile de dilution est choisie parmi les huiles minérales, les huiles d'origine animale, les huiles végétales, les esters d'acides gras et les esters dérivés d'acide lanolique.

12. Emulsion triple selon l'une quelconque des revendications 1 à 10, caractérisée par le fait qu'elle comprend, par rapport au poids total de l'émulsion triple, 0,02 à 0,7% en poids d'émulsionnant(s) E/H, 0,2 à 10% en poids de cire(s) de point de fusion égal ou supérieur à 60°C, 5 à 20% en poids d'huile(s), 0,5 à 20% en poids de cire(s) de point de fusion inférieur à 60°C, 0,1 à 3% de gélifiant à chaîne grasse, des neutralisants, des co-émulsionnants de l'émulsion primaire, ainsi qu'éventuellement des substances actives cosmétologiques ou dermatologiques, et tout autre adjuvant habituellement utilisé en cosmétique, le complément étant constitué par de l'eau.

13. Procédé de préparation d'une émulsion triple eau/huile/eau gélifiée selon l'une quelconque des revendications précédentes, caractérisé par le fait que, dans un premier temps, on réalise l'émulsion primaire E/H en ajoutant une phase aqueuse à une phase grasse contenant au moins une cire de point de fusion égal ou supérieur à 60°C, dans un deuxième temps, on dilue l'émulsion primaire obtenue précédemment avec une huile identique à ou différente de celle utilisée dans l'émulsion primaire et enfin, on réalise l'émulsion triple par addition de l'émulsion primaire diluée obtenue précédemment à une seconde phase aqueuse externe continue gélifiée contenant un gélifiant à chaîne grasse, émulsion triple dans laquelle la quantité de cire de point de fusion égal ou supérieur à 60°C, est comprise entre 0,2 et 10% en poids, et la quantité de phase grasse provenant de l'émulsion E/H est comprise entre 1 et 30% en poids, sur la base du poids total de l'émulsion triple.

14. Procédé selon la revendication 13, caractérisé par le fait qu'on introduit des adjuvants habituellement utilisés en cosmétique et/ou au moins une substance active dans l'une quelconque des phases constitutives de l'émulsion triple ou dans l'émulsion triple finale après sa réalisation.

15. Composition cosmétique destinée à être appliquée sur la peau ou les cheveux, caractérisée par le fait qu'elle comprend une émulsion triple telle que définie dans l'une quelconque des revendications 1 à 12.

16. Composition destinée à être utilisée en dermatologie et contenant des agents dermatologiquement actifs, caractérisée par le fait que son support est constitué par une émulsion triple telle que définie dans l'une quelconque des revendications 1 à 12.

## Claims

1. Gelled water/oil/water triple emulsion, characterized in that it contains:
(A) a fatty phase comprising at least one wax with a melting point above or equal to 60°C, constituting with an aqueous phase the W/O primary emulsion;
(B) a dilution oil;
(C) a gelled continuous external aqueous phase comprising at least one fatty chain-containing gelling agent of the C₃-C₆ monoethylenic carboxylic acid or acid anhydride/fatty chain-containing acrylic ester copolymer type;
in which triple emulsion the amount of fatty phase derived from the W/O emulsion is between 1 and 30% and the amount of wax(es) with a melting point above or equal to 60°C is between 0.2 and 10%, based on the total weight of the triple emulsion.

2. Triple emulsion according to Claim 1, characterized in that the amount of fatty phase in the W/O primary emulsion is greater than 35% by weight.

3. Triple emulsion according to Claim 1 or 2, characterized in that the gelled external aqueous phase additionally contains another gelling agent chosen from carboxyvinyl polymers, polyglyceryl methacrylates, carrageenans and xanthan gums.

4. Triple emulsion according to any one of Claims 1 to 3, characterized in that the gelled external aqueous phase additionally contains glycols, neutralizing agents, preserving agents, dyes, fragrances, active substances, sunscreen agents, moisturizing agents and creaminess agents.

5. Triple emulsion according to any one of Claims 1 to 4, characterized in that the fatty phase of the W/O primary emulsion additionally comprises waxes with a melting point below 60°C such as mineral waxes, waxes of animal origin, lanolin or derivates thereof, hydrogenated oils which are solid at 25°C, mineral oils, oils of animal origin, plant oils, fatty acid esters, esters derived from lanolic acid and fatty alcohols.

6. Triple emulsion according to any one of Claims 1 to 5, characterized in that the aqueous phase of the primary emulsion comprises, in addition to water, moisturizing agents, polyols, active substances, preserving agents, sunscreen agents, fragrances and dyes.

7. Triple emulsion according to any one of Claims 1 to 6, characterized in that the W/O primary emulsion comprises at least one emulsifying agent chosen from anionic surfactants, such as the metal salts or the organic salts of fatty acids containing 12 to 18 carbon atoms, the alkali metal salts or the organic-base salts of alkylsulfuric and alkylsulfonic acids containing 12 to 18 carbon atoms, alkylarylsulfonic acids, the alkyl chain of which contains 6 to 8 carbon atoms, ether sulfates, and polyalkoxylated and/or polyglycerolated nonionic surfactants.

8. Triple emulsion according to any one of Claims 1 to 7, characterized in that the W/O primary emulsion contains a co-emulsifying agent chosen from hydrogenated or unhydrogenated lecithins, and stabilizing inorganic salts.

9. Triple emulsion according to any one of Claims 1 to 8, characterized in that the W/O primary emulsion contains at least one compound containing organofluorine groups and hydrocarbon groups.

10. Triple emulsion according to Claim 9, characterized in that the compound containing organofluorine groups and hydrocarbon groups has a degree of substitution of between 0.5 and 95%.

11. Triple emulsion according to any one of Claims 1 to 10, characterized in that the dilution oil is chosen from mineral oils, oils of animal origin, plant oils, fatty acid esters and esters derived from lanolic acid.

12. Triple emulsion according to any one of Claims 1 to 10, characterized in that it comprises, relative to the total weight of the triple emulsion, 0.02 to 0.7% by weight of W/O emulsifying agent(s), 0.2 to 10% by weight of wax(es) with a melting point above or equal to 60°C, 5 to 20% by weight of oil(s), 0.5 to 20% by weight of wax(es) with a melting point below 60°C, 0.1 to 3% of fatty chain-containing gelling agent, neutralizing agents, co-emulsifying agents for the primary emulsion and optionally cosmetological or dermatological active substances and any other adjuvant usually used in cosmetics, the remainder consisting of water.

13. Process for the preparation of a gelled water/oil/water triple emulsion according to any one of the preceding claims, characterized in that, in a first step, the W/O primary emulsion is prepared by adding an aqueous phase to a fatty phase containing at least one wax with a melting point above or equal to 60°C, in a second step, the primary emulsion obtained above is diluted with an oil which is identical to or different from that used in the primary emulsion and, finally, the triple emulsion is prepared by adding the diluted primary emulsion obtained above to a second gelled continuous external aqueous phase containing a fatty chain-containing gelling agent, in which triple emulsion the amount of wax with a melting point above or equal to 60°C is between 0.2 and 10% by weight, and the amount of fatty phase derived from the W/O emulsion is between 1 and 30% by weight, based on the total weight of the triple emulsion.

14. Process according to Claim 13, characterized in that adjuvants usually used in cosmetics and/or at least one active substance are introduced into any of the phases constituting the triple emulsion or into the final triple emulsion after preparation thereof.

15. Cosmetic composition intended to be applied to the skin or to the hair, characterized in that it comprises a triple emulsion as defined in any one of Claims 1 to 12.

16. Composition intended to be used in dermatology and containing dermatologically active agents, characterized in that the vehicle thereof consists of a triple emulsion as defined in any one of Claims 1 to 12.

## Patentansprüche

1. Gelierte Wasser/Öl/Wasser-Dreifach-Emulsion
dadurch **gekennzeichnet**, daß
sie umfaßt:
(A) eine FettPhase, enthaltend mindestens ein Wachs eines Schmelzpunkts von gleich oder höher als 60°C, welche mit einer wässrigen Phase eine W/O-Primäremulsion bildet;
(B) ein Verdünnungsöl;
(C) eine wässrige äußere kontinuierliche gelierte Phase, enthaltend mindestens ein Geliermittel mit Fett-Kette des Typs eines Copolymer aus monoethylenischer C₃₋₆-Carboxylsäure oder -anhydrid/Acrylester mit Fett-Kette,
wobei in der Dreifach-Emulsion die Menge der Fett-Phase der W/O-Emulsion 1 bis 30% und die Menge an Wachs eines Schmelzpunkts von gleich oder höher als 60°C 0,2 bis 10% betragen, bezogen auf das Gesamtgewicht der Dreifach-Emulsion.

2. Dreifach-Emulsion gemäß Anspruch 1,
dadurch **gekennzeichnet**, daß
die Menge der Fett-Phase in der W/O-Primäremulsion mehr als 35 Gew.% beträgt.

3. Dreifach-Emulsion gemäß Anspruch 1 oder 2,
dadurch **gekennzeichnet**, daß
die wässrige äußere gelierte Phase, zusätzlich, ein weiteres Geliermittel enthält, ausgewählt aus Carboxyvinyl-Polymeren, Glycerylpolymethacrylaten, Karraghenanen und aus Gummiprodukten von Xanthan.

4. Dreifach-Emulsion gemäß einem der Ansprüche 1 bis 3,
dadurch **gekennzeichnet**, daß
die wässrige äußere gelierte Phase ausserdem Glycole, Neutralisiermittel, Konservierungsstoffe, Farbstoffe, Parfüm-Produkte, Wirksubstanzen, Sonnenfilterstoffe, hydratisierende Mittel und Salbungsmittel enthält.

5. Dreifach-Emulsion gemäß einem der Ansprüche 1 bis 4,
dadurch **gekennzeichnet**, daß
die Fett-Phase der W/O-Primäremulsion, zusätzlich, Wachse eines Schmelzpunkts von unterhalb 60°C wie mineralische Wachse, Wachse tierischen Ursprungs, Lanolin oder seine Derivate, hydrierte, bei 25°C feste Öle, mineralische Öle, Öle tierischen Ursprungs, pflanzliche Öle, Fettsäureester, Esterderivate von Lanolinsäure und Fettalkohole enthält.

6. Dreifach-Emulsion gemäß einem der Ansprüche 1 bis 5,
dadurch **gekennzeichnet**, daß
die wässrige Phase der Primäremulsion, ausser Wasser, Hydratisiermittel, Polyole, Aktivsubstanzen, Konservierungsstoffe, Sonnenfilterstoffe, Parfüm-Produkte und Farbstoffe enthält.

7. Dreifach-Emulsion gemäß einem der Ansprüche 1 bis 6,
dadurch **gekennzeichnet**, daß
die W/O-Primäremulsion mindestens einen Emulgator enthält, ausgewählt aus anionischen oberflächenaktiven Mitteln, wie Metallsalzen oder organischen Salzen von Fettsäuren mit 12 bis 18 Kohlenstoffatomen, Alkalisalzen oder Salzen organischer Basen von Alkylschwefel- und Alkylsulfonsäuren mit 12 bis 18 Kohlenstoffatomen, Alkylarylsulfonsäuren mit einer Alkylkette von 6 bis 8 Kohlenstoffatomen, aus Ethersulfaten und aus nicht-ionischen polyalkoxylierten und/oder polyglycerierten oberflächenaktiven Mitteln.

8. Dreifach-Emulsion gemäß einem der Ansprüche 1 bis 7,
dadurch **gekennzeichnet**, daß
die W/O-Primäremulsion einen Coemulgator, ausgewählt aus gegebenenfalls hydrierten Lecithinen, und stabilisierende Mineralsalze enthält.

9. Dreifach-Emulsion gemäß einem der Ansprüche 1 bis 8,
dadurch **gekennzeichnet**, daß
die W/O-Primäremulsion mindestens eine Verbindung enthält, die organofluorierte Kettenglieder und Kohlenwasserstoff-Kettenglieder aufweist.

10. Dreifach-Emulsion gemäß Anspruch 9,
dadurch **gekennzeichnet**, daß
die Verbindung mit organofluorierten und Kohlenwasserstoff-Kettengliedern einen Substitutionsgrad von 0,5 bis 95% aufweist.

11. Dreifach-Emulsion gemäß einem der Ansprüche 1 bis 10,
dadurch **gekennzeichnet**, daß
das Verdünnungsöl aus Mineralölen, Ölen tierischen Ursprungs, pflanzlichen Ölen, Fettsäureestern und aus Esterderivaten von Lanolinsäure ausgewählt ist.

12. Dreifach-Emulsion gemäß einem der Ansprüche 1 bis 10,
dadurch **gekennzeichnet**, daß
sie, bezogen auf das Gesamtgewicht der Dreifach-Emulsion, 0,02 bis 0,7 Gew.% W/O-Emulgator, 0,2 bis 10 Gew.% Wachs eines Schmelzpunkts von gleich oder höher als 60°C, 5 bis 20 Gew.% Öl, 0,5 bis 20 Gew.% Wachs eines Schmelzpunkts von unterhalb 60°C, 0,1 bis 3 Gew.% Geliermittel mit Fett-Kette, Neutralisiermittel, Coemulgatoren für die Primäremulsion sowie gegebenenfalls kosmetologische oder dermatologische Wirksubstanzen und jeden weiteren, gewöhnlich in der Kosmetik verwendeten Hilfsstoff enthält, wobei der Rest aus Wasser zusammengesetzt ist.

13. Verfahren zur Herstellung einer gelierten Wasser/Öl/Wasser-Dreifach-Emulsion gemäß einem der vorhergehenden Ansprüche,
dadurch **gekennzeichnet**, daß
man, in einer ersten Stufe, eine W/O-Primäremulsion herstellt, indem man eine wässrige Phase zu einer Fett-Phase gibt, die mindestens ein Wachs eines Schmelzpunkts von gleich oder höher als 60°C enthält, daß man, in einer zweiten Stufe, die vorher erhaltene Primäremulsion mit einem Öl verdünnt, das dem in der Primäremulsion verwendeten Öl gleicht oder davon verschieden ist, und daß man schließlich die Dreifach-Emulsion herstellt, indem man die vorher erhaltene verdünnte Primäremulsion zu einer wässrigen äußeren kontinuierlichen gelierten zweiten Phase gibt, die ein Geliermittel mit Fett-Kette enthält, wobei in der Dreifach-Emulsion die Menge an Wachs eines Schmelzpunkts von gleich oder höher als 60°C 0,2 bis 10 Gew.% und die Menge an Fett-Phase der W/O-Emulsion 1 bis 30 Gew.% betragen, bezogen auf das Gesamtgewicht der Dreifach-Emulsion.

14. Verfahren gemäß Anspruch 13,
dadurch **gekennzeichnet**, daß
man gewöhnlich in der Kosmetik eingesetzte Hilfsstoffe und/oder mindestens eine Wirksubstanz in eine der die Dreifach-Emulsion bildenden Phasen oder in die endgültige Dreifach-Emulsion nach ihrer Herstellung einbringt.

15. Kosmetische Zusammensetzung zur Aufbringung auf die Haut oder die Haare,
dadurch **gekennzeichnet**, daß
sie eine in einem jeden der Ansprüche 1 bis 12 definierte Dreifach-Emulsion enthält.

16. Zusammensetzung zur Verwendung in der Dermatologie, die dermatologisch wirksame Mittel enthält,
dadurch **gekennzeichnet**, daß
ihre Trägergrundlage aus einer in einem jeden der Ansrpüche 1 bis 12 definierten Dreifach-Emulsion zusammengesetzt ist.
